# EUROPEAN PATENT APPLICATION

(11) **EP 3 721 730 A1**
(43) Date of publication of application: **14.10.2020**
(21) Application number: 19169056.9
(22) Date of filing: 12.04.2019
(51) Int. Cl.: A24F 47/00

(54) **A PROTECTIVE MEMBER FOR A SMOKING SUBSTITUTE DEVICE**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

Various embodiments provide a protective member for a smoking substitute device, the protective member comprising a retainer for receiving the smoking substitute device, the retainer being configured to frictionally engage with a surface of the smoking substitute device to secure the protective member to the smoking substitute device. Other embodiments provide a smoking substitute kit comprising a protective member and a smoking substitute device.

## Description

### Field of the Invention

The present invention relates to a protective member for a smoking substitute device.

### Background

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Conventional combustible smoking articles, such as cigarettes, typically comprise a cylindrical rod of tobacco comprising shreds of tobacco which is surrounded by a wrapper, and usually also a cylindrical filter axially aligned in an abutting relationship with the wrapped tobacco rod. The filter typically comprises a filtration material which is circumscribed by a plug wrap. The wrapped tobacco rod and the filter are joined together by a wrapped band of tipping paper that circumscribes the entire length of the filter and an adjacent portion of the wrapped tobacco rod. A conventional cigarette of this type is used by lighting the end opposite to the filter, and burning the tobacco rod. The smoker receives mainstream smoke into their mouth by drawing on the mouth end or filter end of the cigarette.

Combustion of organic material such as tobacco is known to produce tar and other potentially harmful byproducts. There have been proposed various smoking substitute devices (or "substitute smoking devices") in order to avoid the smoking of tobacco.

Such smoking substitute devices can form part of nicotine replacement therapies aimed at people who wish to stop smoking and overcome a dependence on nicotine.

Smoking substitute devices may comprise electronic systems that permit a user to simulate the act of smoking by producing an aerosol, also referred to as a "vapour" that is drawn into the lungs through the mouth (inhaled) and then exhaled. The inhaled aerosol typically bears nicotine and/or flavourings without, or with fewer of, the odour and health risks associated with traditional smoking.

Some smoking substitute devices are designed to resemble a traditional cigarette and are cylindrical in form with a mouthpiece at one end. Other smoking substitute devices do not generally resemble a cigarette (for example, the smoking substitute device may have a generally box-like form).

There are a number of different categories of smoking substitute systems, each utilising a different smoking substitute approach.

One approach is the so-called "vaping" approach, in which a vaporisable liquid, typically referred to (and referred to herein) as "e-liquid", is heated by a heating device (referred to herein as an electronic cigarette or "e-cigarette" device) to produce an aerosol vapour which is inhaled by a user. The e-liquid typically includes a base liquid as well as nicotine and/or a flavourant. The resulting vapour therefore also typically contains nicotine and/or a flavourant. The base liquid may include propylene glycol and/or vegetable glycerine.

A typical e-cigarette device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

E-cigarettes can be configured in a variety of ways. For example, there are "closed system" vaping smoking substitute systems, which typically have a sealed tank and heating element. The tank is prefilled with e-liquid and is not intended to be refilled by an end user. One subset of closed system vaping smoking substitute systems include a main body which includes the power source, wherein the main body is configured to be physically and electrically coupled to a consumable including the tank and the heating element. In this way, when the tank of a consumable has been emptied, that consumable is disposed of. The main body can be reused by connecting it to a new, replacement, consumable. Another subset of closed system vaping smoking substitute systems are completely disposable, and intended for one-use only.

There are also "open system" vaping smoking substitute systems which typically have a tank that is configured to be refilled by a user. In this way the entire device can be used multiple times.

An example vaping smoking substitute device is the myblu™ e-cigarette. The myblu™ e-cigarette is a closed system device which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heating device, which for this device is a heating filament coiled around a portion of a wick. The wick is partially immersed in the e-liquid, and conveys e-liquid from the tank to the heating filament. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

Another approach for a smoking substitute device is the so-called "heat not burn" ("HNB") approach in which tobacco (rather than e-liquid) is heated or warmed to release vapour. The tobacco may be leaf tobacco or reconstituted tobacco. The vapour may contain nicotine and/or flavourings. In the HNB approach the intention is that the tobacco is heated but not burned, i.e. the tobacco does not undergo combustion.

A typical HNB smoking substitute device may include a main body and a consumable. The consumable may include the tobacco material. The main body and consumable may be configured to be physically coupled together. In use, heat may be imparted to the tobacco material by a heating device that is typically located in the main body, wherein airflow through the tobacco material causes moisture in the tobacco material to be released as vapour. A vapour may be formed from a carrier in the tobacco material (this carrier may for example include propylene glycol and/or vegetable glycerine) and additionally volatile compounds released from the tobacco. The released vapour may be entrained in the airflow drawn through the tobacco.

As the vapour passes through the smoking substitute device (entrained in the airflow) from an inlet to a mouthpiece (outlet), the vapour cools and condenses to form an aerosol (also referred to as a vapour) for inhalation by the user. The aerosol will normally contain the volatile compounds.

In general, smoking substitute devices are intended to provide a substitute for the rituals of smoking, whilst providing the user with a similar experience and satisfaction to those experienced with traditional smoking and with combustible tobacco products.

The popularity and use of smoking substitute devices has grown rapidly in the past few years. Although originally marketed as an aid to assist habitual smokers wishing to quit tobacco smoking, consumers are increasingly viewing smoking substitute devices as desirable lifestyle accessories. As lifestyle accessories, some users may find it desirable to personalise or customise their smoking substitute device, for example to help distinguish their own device from other, similar devices.

In general, smoking substitute devices are not disposable, and it is intended that a user will keep the same device for a long time. For example, in either an "open system" or "closed system" smoking substitute device, the intention is that a user will retain and reuse the main body of the smoking substitute device. Users may therefore desire to prolong the lifespan of their device, as it is cheaper and easier to replace e-liquid or consumables than it is to repair or replace the main body of the device.

The present invention has been devised in light of the above considerations.

### Summary of the Invention

At its most general, the present invention relates to a protective member for a smoking substitute device. In this way, the protective member may be fitted to the smoking substitute device in order to protect the device, for example, from damage during use.

According to a first aspect of the present invention, there is provided a protective member for a smoking substitute device, the protective member comprising a retainer for receiving the smoking substitute device, the retainer being configured to frictionally engage with a surface of the smoking substitute device to secure the protective member to the smoking substitute device. The retainer may be referred to herein as a jacket, sleeve, cover, holder or casing for a smoking substitute device. The protective member may thereby protect the smoking substitute device, for example if the device is dropped, and so prolong the lifespan of the device. The protective member may be supplied in a variety of colours, styles and/or materials such that by using a protective member according to the present invention a user is provided with a degree of customisability or personalisation for their smoking substitute device. An outer surface of the protective member may also be provided with a logo, design, picture, or pattern which may be desirable for a user. For example, such pictures or patterns may be applied to the surface by painting, or as a sticker. In other examples, the outer surface of the protective member may be engraved. Different configurations of the protective member may also increase comfort for the user in using the smoking substitute device, for example making the device easer to hold and therefore easier and more pleasurable to use. For example, the protective member may be more haptically pleasing to a user than the main body of a smoking substitute device. The protective member may also provide hygiene advantages, for example it may stop certain parts of the smoking substitute device, such as a mouthpiece, from coming into contact with surfaces upon which the device is placed when not in use. It is to be understood that the protective member is releasably secured to the smoking substitute device such that it can be removed from the smoking substitute device.

Optionally, the retainer comprises a sleeve, wherein the retainer is configured to receive the smoking substitute device in a bore defined by an interior of the sleeve . The term "bore" may generally refer to a void, cavity or space which is defined by an interior of the sleeve. For example, where the sleeve is tubular, the bore may be elongate and/or cylindrical in shape. However, in some other embodiments, the sleeve and bore may have a non-circular cross-section, such as, for example, oval, square, rectangular or irregular. Preferably, the cross-section of the bore is chosen to match the shape of a particular make or model of substitute smoking device, such as the myblu™ e-cigarette, for example, the bore may have a truncated-oval cross-section. The sleeve may be less than 100mm in length, such as 90mm, 80mm, 70mm, 60mm, 50mm, 40mm, 30mm, 20mm, 10mm or less. In some embodiments, a width of the sleeve is greater than or equal to a length of the sleeve. In this way, the sleeve may be substantially annular in shape, and is configured to cover a minority of a body portion of the smoking substitute device.

Preferably, the retainer further comprises a second sleeve, where the retainer is configured to receive the smoking substitute device in a bore defined by an interior of the second sleeve; and a web portion connecting the sleeve and the second sleeve together in an axially (or longitudinally) spaced relationship along a longitudinal axis of the protective member. For example, an interior surface of the web portion may frictionally engage with a surface of the smoking substitute device. The second sleeve may be less than 100mm in length, such as 90mm, 80mm, 70mm, 60mm, 50mm, 40mm, 30mm, 20mm, 10mm or less. A protective member having a second sleeve may provide sufficient protection for the smoking substitute device in a lightweight design, making the protective member more ergonomic for a user.

In some embodiments, the sleeve may be closed at one end, which may help to prevent the smoking substitute device from slipping from within the protective member.

Conveniently, the retainer comprises two elongate arms which are spaced apart from one another along their length, wherein the retainer is configured to receive the smoking substitute device in the region defined between the two elongate arms. Preferably the two elongate arms may be joined together at a respective first end, for example by a web portion which may help to prevent the smoking substitute device from slipping from between the two elongate arms. Additionally or alternatively, the two elongate arms may be joined together along a portion (e.g. a majority or minority) of their respective lengths, for example by at least one web portion which is arranged to frictionally engage with either a front or back of the smoking substitute device. Advantageously, each of the two elongate arms may have a generally u-shaped cross section such that each arm is configured to receive an edge portion of the smoking substitute device.

Preferably, the protective member comprises a window for viewing a surface of the smoking substitute device. For example, the window may comprise an aperture or notch through a sidewall of the protective member. One or more windows may be provided in the protective member in this way for aesthetics, for example for personalisation of the smoking substitute device. Additionally or alternatively, apertures may be provided in the protective member to aid use of the smoking substitute device, for example to allow a user to see visual indicators (e.g. a light or an e-liquid tank window) which are provided as part of the smoking substitute device. Apertures in the protective member may also allow access to ports in the main body of a smoking substitute device, such as a charging port. For example, a particular smoking substitute device may comprise a USB port for charging and for data transfer, and the protective member may comprise a corresponding aperture to allow access to such a port while the protective member is positioned on the main body of the smoking substitute device.

Optionally, the protective member comprises a grippy material, which is a material which ensures a high coefficient of friction between the protective member and the surface of the smoking substitute device. This may help ensure that the protective member stays in position with respect to the body portion of the smoking substitute device (e.g. when the grippy material is on an inner surface of the protective member), and may also help a user hold the smoking substitute device (e.g. when the grippy material is on an outer surface of the protective member). Additionally or alternatively, the protective member may be made of a resilient material so as to provide shock-absorbing properties which helps to protect the smoking substitute device if it is dropped by a user, for example. For example, the protective member may be made of silicone, which provides both resilient and high-friction properties for these purposes, though any other suitable material may be chosen, such as any rubber-like material. In some embodiments, the protective member may comprise two or more different materials. For example, the protective member may be coated or otherwise covered in a material which is chosen for its haptically pleasing or visually pleasing properties.

Advantageously, the protective member may comprise a gripping feature on an outer surface thereof. A gripping feature provided in this way helps a user to hold the smoking substitute device when the protective member is use. For example, the gripping feature may comprise groove on an outer surface of the protective member. Additionally or alternatively, the protective member may comprise a ridge on an outer surface thereof. These features may improve the ergonomics and comfort of the protective member, by providing convenient locations for a user to position a finger or thumb, which may also help a user to grip the smoking substitute device. For example, the protective member may comprise a plurality of grooves and/or ridges in an outer surface thereof which may increase friction between a user's finger and the protective member.

According to a second aspect of the invention, there is provided a smoking substitute device; and a protective member according to the first aspect of the invention. Advantageously, a shape of the retainer substantially matches a cross-sectional shape of the smoking substitute device, thereby ensuring that the protective member may be properly secured to the smoking substitute device. For example, the shape and dimensions of the retainer may be selected such that the retainer forms an interference fit with the smoking substitute device. That is, the shape of the retainer may be complementary to the shape of the smoking substitute device.

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
**Figure 1(a)** shows an example smoking substitute device, **Figure 1(b)** shows the main body of the smoking substitute device without the consumable, **Figure 1(c)** shows the consumable of the smoking substitute device without the main body;
**Figure 2(a), Figure 2(b), and Figure 2(c)** each show a protective member according to an embodiment of the present invention; and
**Figure 3(a), Figure 3(b), and Figure 3(c)** each show a protective member according to an embodiment of the present invention.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

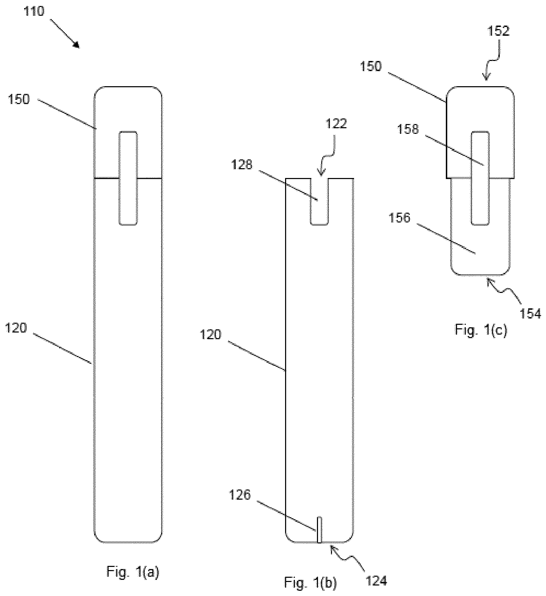

Fig. 1(a) shows an example smoking substitute device 110 for use as a smoking substitute device.

In this example, the smoking substitute device 110 includes a main body 120 and a consumable 150. The consumable 150 may alternatively be referred to as a "pod".

In this example, the smoking substitute device 110 is a closed system vaping device, wherein the consumable 150 includes a sealed tank 156 and is intended for one-use only.

Fig. 1(a) shows the smoking substitute device 110 with the main body 120 physically coupled to the consumable 150.

Fig. 1(b) shows the main body 120 of the smoking substitute device 110 without the consumable 150.

Fig. 1(c) shows the consumable 150 of the smoking substitute device 110 without the main body 120.

The main body 120 and the consumable 150 are configured to be physically coupled together, in this example by pushing the consumable 150 into an aperture in a top end 122 of the main body 120. In other examples, the main body 120 and the consumable could be physically coupled together by screwing one onto the other, or through a bayonet fitting, for example. An optional light 126, e.g. an LED located behind a small translucent cover, is located a bottom end 124 of the main body 120. The light 126 may be configured to illuminate when the smoking substitute device 110 is activated.

The consumable 150 includes a mouthpiece (not shown) at a top end 152 of the consumable 150, as well as one or more air inlets (not shown in Fig. 1) so that air can be drawn into the smoking substitute device 110 when a user inhales through the mouthpiece. At a bottom end 154 of the consumable 150, there is located a tank 156 that contains e-liquid. The tank 156 may be a translucent body, for example.

The tank 156 preferably includes a window 158, so that the amount of e-liquid in the tank 156 can be visually assessed. The main body 120 includes a slot 128 so that the window 158 of the consumable 150 can be seen whilst the rest of the tank 156 is obscured from view when the consumable 150 is inserted into the aperture in the top end 122 of the main body 120.

The tank 156 may be referred to as a "clearomizer" if it includes a window 158, or a "cartomizer" if it does not.

In the illustrated embodiment, the consumable 150 is a "single-use" consumable 150. That is, upon exhausting an e-liquid from the consumable 150, the intention is that the user disposes of the entire consumable 150. In other embodiments, the e-liquid (i.e. aerosol former) may be the only part of the system that is truly "single-use". That is, the tank may be refillable with e-liquid or the e-liquid may be stored in a non-consumable component of the system. For example, the e-liquid may be stored in a tank located in the main body or stored in another component that is itself not single-use (e.g. a refillable cartomizer).

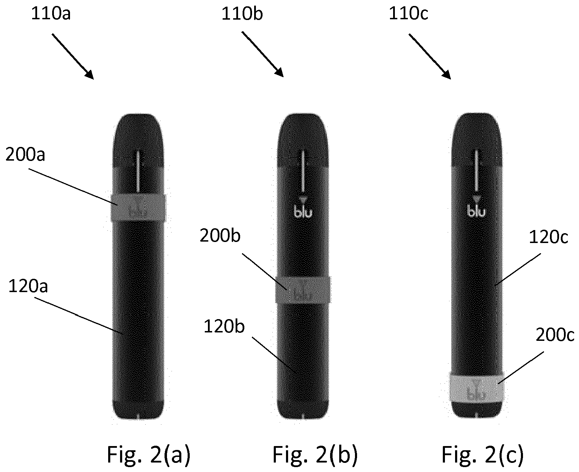

The main body 120 may comprise a power source of in the form of a battery (e.g. a rechargeable battery). The main body 120 may comprise a connector in the form of e.g. a USB port for recharging this battery. The main body 120 may also comprise a controller that controls the supply of power from the power source to the main body electrical contacts. In this way, the smoking substitute system 110 may only activate under certain conditions (e.g. during a puff and/or only when the system is in an active state Figs. 2(a)-2(c) show example protective members 200a, 200b, 200c positioned on a body portion 120a, 120b, 120c of a smoking substitute device 110a, 110b, 110c. The smoking substitute devices 110a, 110b, 110c are substantially the same as that described above with respect to Fig. 1. In these examples, each protective member 200 comprises a generally annular retainer which frictionally engages the outer surface of the smoking substitute device 110a, 110b, 110c. The retainer is sized to fit around the entire circumference of the body portion 120, but has a length which is shorter than its width such that the retainer covers a minority of the smoking substitute device 110a, 110b, 110c. The protective member 200 is held in place by frictional engagement with the surface of the body portion 120. The cross-section of the protective member 200 is shaped to closely match or conform to the cross-section of the main body of the smoking substitute device 110 to ensure a good frictional engagement such that the protective member 200 is held in place until a user moves it. To further aid this frictional engagement, the protective member 200 is preferably made of a grippy material which increases friction between the protective member 200 and the body portion 120, and thus hold the protective member 200 in a position which is desired by a user. For example, the protective member 200 may be made of silicone or another rubber-like or tacky material.

The protective members 200a, 200b, 200c serve a number of purposes which a user may find desirable. For example, a protective member 200 may be made of a shock-absorbing material to help avoid damage to the smoking substitute device 110 if it is dropped. The shock-absorbing material may be silicone, for example, but another rubber-like or resilient material may be used. As mentioned above, the protective member 200 may preferably be made of a material which increases friction. As well as increasing friction between the protective member 200 and the body portion 120, such materials also make it easier for a user to grip the protective member 200 and thereby also grip the smoking substitute device 110. This may reduce the risk of a user dropping the smoking substitute device 110. Other materials may be chosen to comprise at least a portion of the protective member 200. For example, the protective member 200 may comprise materials which a user will find haptically pleasing (e.g. silicone or leather). The protective member 200 may be made largely or entirely of such materials, or these materials may be used only as a coating.

As the protective member 200 is an annular structure, with a length which is substantially less than the length of the body portion 120, a user may position the protective member 200 on the body portion 120 at any desired location, as shown in Figs. 2(a)-(c). The user may thereby position the protective member 200 at a desired location for a more ergonomic grip of the smoking substitute device 120, or may choose to position two or more protective members 200 on a given smoking substitute device. In this way, the protective member 200 provides a degree of customisability to the smoking substitute device 120. Customisability may also be enhanced by providing the protective member 200 in a variety of materials as mentioned above, and/or any desired colour. For example, the protective member 200 may be supplied in a limited number of colours to match the branding of the smoking substitute device. In some embodiments, the protective member 200 may be patterned.

As shown in Fig. 2(a) the protective member 200a may be positioned towards a mouthpiece of the smoking substitute device 110a, for example, at an end of the body portion 120a which is close to the consumable 150. By positioning the protective member 200a in this way, the protective member 200a may act to raise a mouthpiece of the smoking substitute device 110a from a surface on which the smoking substitute device 110a may be placed when it is not in use by a user. This may ensure that the mouthpiece of the smoking substitute device 110a is kept clean, and is not soiled by contact with the surface.

An image, logo, or text may be printed, engraved, embossed or otherwise displayed on the outer surface of the protective member 200. For example, the protective member 200 may carry branding to match branding present on the smoking substitute device 110. This may provide a further degree of customisability for a user.

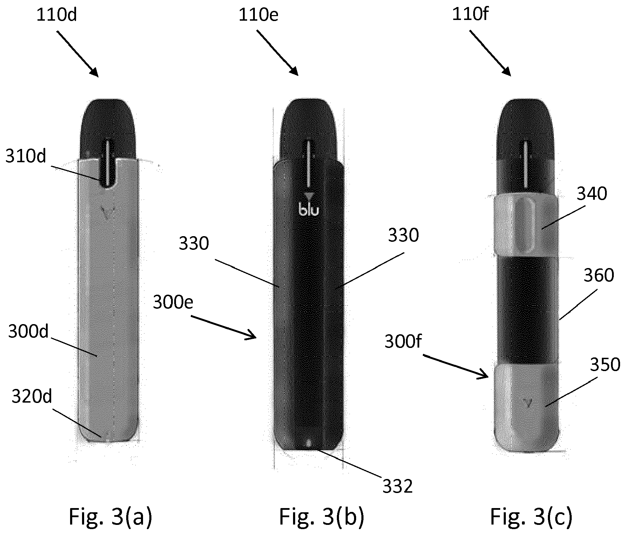

Figs. 3(a)-3(c) show three further example protective members 300d, 300e, 300f positioned on a body portion of a respective smoking substitute device 110d, 110e, 110f. The smoking substitute devices 110d, 110e, 110f are substantially as described with respect to Fig. 1. The protective members 300d, 300e, 300f share many features and advantages with the protective members 200a, 200b, 200c discussed above with relation to Figs. 2(a)-2(c). For example, each protective member 300 may be made of a rubber-like material in order to increase friction, which may help ensure that the protective member 300 does not separate from the smoking substitute device 110 and also helps a user hold the smoking substitute device 110. Preferably, the material may also have shock-absorbing properties in order to protect the smoking substitute device 110, for example if it is dropped. Silicone may be a particularly suitable material for the protective member 300. As the protective members 300d, 300e, 300f cover a large proportion (e.g. a majority) of the smoking substitute device 110, they are also suitable for raising the mouthpiece of the smoking substitute device 110 from a surface as described above with respect to Fig. 2(a). Each protective member 300d, 300e, 300f may provide customisability for a smoking substitute device 110 by being supplied in a variety of materials, colours and/or patterns, and by providing a surface for text, images or logos as required. In these examples, the protective members 300d, 300e, 300f cover a larger proportion (e.g. a majority) of the smoking substitute device 110 compared to the protective members 200a, 200b, 200c, which cover a smaller proportion (e.g. only a minority) of the smoking substitute device 110.

Fig. 3(a) shows a protective member 300d positioned on a body portion of a smoking substitute device 110d. The protective member 300d has a retainer comprising a sleeve, wherein the sleeve is a generally tubular structure which is dimensioned to fit over substantially all of the body portion of the smoking substitute device 110d, where it is held in place by frictional engagement with the surface of the body portion. The sleeve has an interior bore for receiving the smoking substitute device, wherein the cross-section of the bore may be chosen to match or closely conform to the cross-sectional shape of the main body of the smoking substitute device 110d to ensure good frictional engagement (e.g. an interference fit). For example, the sleeve and bore may have a substantially oval cross-section; however, they may also have cross-sections of other shapes. The protective member 300d has a notch 310d at its first end to allow a user to view the amount of e-liquid remaining in the tank of the consumable of the smoking substitute device 110d. In this way, the notch 310d may be considered a window of the protective member 300d. The user therefore does not need to remove the protective member 300d to accurately gauge how much e-liquid is remaining. The protective member 300d also has an aperture 320d in a second end, opposite the first end, to allow a user to see the light located at the bottom end of the main body of the smoking substitute device (as shown in Fig. 1(b)). In this way, the aperture 320d may also be considered a window of the protective member 300d

Fig. 3(b) shows a protective member 300e positioned on a body portion of a smoking substitute device 110e. In this embodiment, the protective member 300e comprises a retainer having two elongate arms 330, wherein the smoking substitute device 110e is received in the region between the two elongate arms 330. In particular, the elongate arms 330 have a generally u-shaped cross-section in order to engage respective edge portions of the main body of the smoking substitute device 110e, thereby providing protection for the main body. The elongate arms 330 leave the front and rear of the main body of the smoking substitute device 110e substantially uncovered. This allows the user to view the amount of e-liquid remaining in the tank of the consumable, and view the light located at the bottom end of the main body of the smoking substitute device 110e. The elongate arms 330 are joined together at their lower end by a web portion 332 to cover the bottom end of the main body of the smoking substitute device 110e. By being arranged in this way, the protective member 300e may provide protection for the smoking substitute device, as well as comfort and customisability for a user, but at a reduced weight compared with the protective member 300d shown in Fig. 3(a). In the embodiment of Fig. 3(b), the web portion 332 is generally planar and covers only a bottom face of the main body of the smoking substitute device 110e. However, in another embodiment, the web portion 332 may have a generally cap-shape or cup-shape such that the web portion 332 extends part way up the sidewalls of the main body. In this case, the web portion 332 may comprise an aperture 320e to allow a user to see the light located at the bottom end of the main body of the smoking substitute device 110e. Additionally or alternatively, the two elongate arms 330 may be joined together along a portion (e.g. a majority or minority) of their respective lengths on only one side (i.e. a front or a back), for example by at least one web portion (not shown) which is arranged to frictionally engage with (either a front or back of) the smoking substitute device 110e. For example, the at least one web portion may be strap-like and be arranged horizontally with respect to the smoking substitute device 110e.

Fig. 3(c) shows a protective member 300f positioned on a body portion of a smoking substitute device 110f. In this embodiment, the protective member 300f comprises a retainer having an upper sleeve 340 and a lower sleeve 350 connected by a central web section 360. The upper sleeve 340 and lower sleeve 350 each have a bore, with a cross-section shape chosen to closely fit the main body of the smoking substitute device 110f, such that the protective member 300f is held in place by frictional engagement with the surface of the main body. The central web section 360 is designed to leave a portion of the main body of the smoking substitute device 110f uncovered, that is, the web section 360 does not cover the whole circumference of the main body and may only cover a minority of the circumference. By being arranged in this way, the protective member 300f may provide protection for the smoking substitute device, as well as comfort and customisability for a user, but at a reduced weight compared with the protective member 300d shown in Fig. 3(a). In the embodiment depicted in Fig. 3(c) the lower sleeve 350 does not comprise an aperture. However, some examples may provide an aperture to allow a user to see the light located at the bottom end of the main body of the smoking substitute device 110f. The upper sleeve 340 comprises a gripping feature in the form of a groove or indentation which may be suitable to receive the thumb or finger of a user, which helps the user grip the smoking substitute device 110f and may provide additional comfort when the user is using the smoking substitute device 110f.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. A protective member for a smoking substitute device, the protective member comprising a retainer for receiving the smoking substitute device, the retainer being configured to frictionally engage with a surface of the smoking substitute device to secure the protective member to the smoking substitute device.

2. The protective member of claim 1, wherein the retainer comprises a sleeve, and wherein the retainer is configured to receive the smoking substitute device in a bore defined by an interior of the sleeve.

3. The protective member of claim 2, wherein a width of the sleeve is greater than or equal to a length of the sleeve.

4. The protective member of claim 2 or claim 3, wherein the retainer further comprises:
a second sleeve, where the retainer is configured to receive the smoking substitute device in a bore defined by an interior of the second sleeve; and
a web portion connecting the sleeve and the second sleeve together in an axially spaced relationship along a longitudinal axis of the protective member.

5. The protective member of any one of claims 2 to 4, wherein the sleeve is closed at one end.

6. The protective member of claim 1, wherein the retainer comprises two elongate arms which are spaced apart from one another along their length, wherein the retainer is configured to receive the smoking substitute device in a region defined between the two elongate arms.

7. The protective member of claim 6, wherein the two elongate arms are joined together at a respective first end by a web portion, and wherein the web portion defines a base of the retainer.

8. The protective member of claim 6 or claim 7, wherein each of the two elongate arms has a generally u-shaped cross-section, such that each of the two elongate arms is configured to receive an edge portion of the smoking substitute device.

9. The protective member of any preceding claim, wherein the protective member comprises a window for viewing a surface of the smoking substitute device.

10. The protective member of claim 9, wherein the window comprises an aperture or notch through a sidewall of the protective member.

11. The protective member of any preceding claim, wherein the protective member is made from a grippy material.

12. The protective member of any preceding claim, wherein the protective member comprises a gripping feature on an outer surface thereof.

13. The protective member of any preceding claim, wherein the protective member is made from a resilient material.

14. A smoking substitute kit comprising:
a smoking substitute device; and
a protective member according to any preceding claim.

15. The smoking substitute kit of claim 14, wherein a shape of the retainer substantially matches a cross-sectional shape of the smoking substitute device.
